# EUROPEAN PATENT APPLICATION

(11) **EP 4 275 683 A1**
(43) Date of publication of application: **15.11.2023**
(21) Application number: 22736906.3
(22) Date of filing: 10.01.2022
(51) Int. Cl.: A61K 31/37, A61P 31/12, A61P 31/14

(54) **ANTIVIRAL PHARMACEUTICAL COMPOSITION COMPRISING STEROID SULFATASE INHIBITOR**

(30) Priority: 08.01.2021 KR 20210002556
(71) Applicant: Industry-Academic Cooperation Foundation, Yonsei University, Seoul 03722 (KR); Nexyon Biotech Co., Ltd., Seoul 07985 (KR)
(72) Inventor: SEO, Jung Taek, Seoul 04201 (KR); MOON, Seok Jun, Seoul 03703 (KR); KIM, Sung-Jin, Seoul 04734 (KR); LEE, Jae Myun, Seoul 06547 (KR); PARK, Pil-Gu, Seoul 03722 (KR); HWANG, Su Jin, Seoul 04592 (KR); LEE, Moon Geon, Seoul 07247 (KR)
(74) Representative: Müller, Christian Stefan Gerd
(86) International application number: PCT/KR2022/000360
(87) International publication number: WO 2022/149929

(57) **Abstract**

A steroid sulfatase inhibitor provided by the present invention is a safe substance without toxicity and adverse effects, has inhibitory activity against various viruses, and thus is capable of effectively preventing, ameliorating, or treating viral infections or diseases caused by viral infections.

## Description

### Technical Field

The present invention relates to the antiviral use of a steroid sulfatase inhibitor, and furthermore, to the use of the steroid sulfatase inhibitor for preventing, ameliorating or treating viral infection or infectious disease.

### Background Art

Viruses have DNA or RNA as their genome and have a structure surrounded by proteins. Viruses cannot multiply by themselves, and thus they replicate in host cells and multiply through intercellular infection. Viruses are small organisms having both animate and inanimate characteristics, and may infect the human body, causing viral diseases. Since viruses are different from bacteria and their multiplication is not inhibited by conventional antibiotics, viral infections are difficult to treat with conventional antibiotics, and it is necessary to use antiviral agents which are substances that inhibit the multiplication of viruses. Currently, antiviral agents are drugs for weakening the action of viruses that invaded the body or for treating infectious diseases caused by viruses, and antiviral agents developed to date include virus-neutralizing antibodies that neutralize the ability of viruses to infect host cells, and aphidicolin which inhibits the activity of DNA polymerase α involved in viral DNA synthesis. In addition to treatment strategies that inhibit viral penetration and replication, many strategies to overcome viral infection by appropriately controlling the body's immune response, like interferons, have also been attempted.

Recently, new drug development has required a long time, a lot of money and huge efforts. Thus, in order to shorten the new drug development period to the maximum possible extent, many studies on drug repurposing have been conducted with a focus on drugs that prevent viral multiplication by inhibiting the action of viral protein scissors or RNA polymerase, among existing therapeutic agents against RNA viruses (AIDS virus, Ebola virus, flu virus, etc.). Efforts have been made to quickly find substances that have antiviral effects while being safe for host cells, among other therapeutic drugs that have been used in patients or new drug candidates under development. However, to date, there is a lack of drugs that are perfectly safe for host cells, have few side effects, and act effectively.

The present inventors have found that existing therapeutic drugs, which have proven safety and may be used for the treatment of obesity and metabolic diseases, have antiviral effects and may be used as antiviral drugs that do not cause side effects in the human body, thereby completing the present invention.

### DISCLOSURE

### Technical Problem

An object of the present invention is to provide a composition having an antiviral effect against various viruses.

Another object of the present invention is to provide a composition for various uses capable of preventing, ameliorating or treating viral infection or various diseases caused by viral infection.

Still another object of the present invention is to provide a method for inhibiting the activity of various viruses and a method capable of preventing, ameliorating or treating viral infection or various diseases caused by viral infection.

However, objects to be achieved by the present invention are not limited to the above-mentioned objects, and other objects not mentioned herein will be clearly understood by those skilled in the art from the following description.

### Technical Solution

Hereinafter, various embodiments described herein will be described with reference to figures. In the following description, numerous specific details are set forth, such as specific configurations, compositions, and processes, etc., in order to provide a thorough understanding of the present invention. However, certain embodiments may be practiced without one or more of these specific details, or in combination with other known methods and configurations. In other instances, known processes and preparation techniques have not been described in particular detail in order to not unnecessarily obscure the present invention. Reference throughout this specification to "one embodiment" or "an embodiment" means that a particular feature, configuration, composition, or characteristic described in connection with the embodiment is included in at least one embodiment of the present invention. Thus, the appearances of the phrase "in one embodiment" or "an embodiment" in various places throughout this specification are not necessarily referring to the same embodiment of the present invention. Additionally, the particular features, configurations, compositions, or characteristics may be combined in any suitable manner in one or more embodiments.

Unless otherwise stated in the specification, all the scientific and technical terms used in the specification have the same meanings as commonly understood by those skilled in the technical field to which the present invention pertains.

One embodiment of the present invention is directed to an antiviral pharmaceutical composition comprising a steroid sulfatase inhibitor as an active ingredient.

In the present invention, the "steroid sulfatase" serves to regulate the local production of estrogens and androgens from precursors in several tissues. This enzyme is known to play a catalytic role in the process of hydrolyzing the sulfate esters of 3-hydroxy steroids, which are inactive transport or precursor forms of the active 3-hydroxy steroids.

In the present invention, the "steroid sulfatase inhibitor" has the antiviral effect, and thus serves to inhibit the multiplication of viruses.

In the present invention, the steroid sulfatase inhibitor may be at least one selected from the group consisting of irosustat, 2-(hydroxyphenyl) indole sulfate, 5-androstene-3,17-diol-3 sulfate, estrone-3-O-methylthiophosphonate (E1-3-MTP), estrone-3-O-sulfamate (EMATE), estradiol-3-O-sulfamate (E2MATE), 4-methylcoumarin 7-O-sulfamate (COUMATE), KW-2581, STX213, and morpholino, and may be, for example, irosustat, without being limited thereto.

In the present invention, the irosustat may have a structure represented by Formula 1 below, and the IUPAC name of the compound is (6-oxo-8,9,10,11-tetrahydro-7H-cyclohepta[c]chromen-3-yl)sulfamate, and the CAS number thereof may be 288628-05-7.

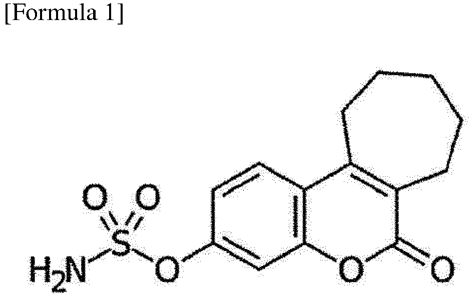

The steroid sulfatase inhibitor provided in the present invention may comprise, for example, a pharmaceutically acceptable salt form of irosustat. The pharmaceutically acceptable salt should be less toxic to the human body and should not adversely affect the biological activity and physicochemical properties of the mother compound. The pharmaceutically acceptable salt may be an acid addition salt of the steroid sulfatase inhibitor, preferably an irosustat compound, which is formed with a pharmaceutically acceptable free acid, without being limited thereto.

A preferred salt form of the compound according to the present invention may be a salt with an inorganic acid or an organic acid. In this case, the inorganic acid may be hydrochloric acid, sulfuric acid, nitric acid, phosphoric acid, perchloric acid, bromic acid, or the like. In addition, the organic acid may be acetic acid, methanesulfonic acid, ethanesulfonic acid, p-toluenesulfonic acid, fumaric acid, maleic acid, malonic acid, phthalic acid, succinic acid, lactic acid, citric acid, gluconic acid, tartaric acid, salicylic acid, malic acid, oxalic acid, benzoic acid, embonic acid, aspartic acid, glutamic acid, or the like. An organic base that may be used to produce an organic base addition salt is tris(hydroxymethyl)methylamine, dicyclohexylamine, or the like. An amino acid that may be used to produce an amino acid addition salt is a naturally occurring amino acid such as alanine, glycine, or the like. It will be apparent to those skilled in the art that acids or bases other than the above-described inorganic acids, organic acids, organic bases, and amino acids may be used.

In the present invention, the salt form may be produced by a conventional method. For example, the salt form may be produced by dissolving the steroid sulfatase inhibitor, for example, irosustat, in a water-miscible solvent such as methanol, ethanol, acetone, or 1,4-dioxane, and then adding a free acid or base thereto, followed by crystallization.

Meanwhile, in addition to the compounds according to the present invention, a hydrate or solvate form of the steroid sulfatase inhibitor, for example, irosustat, may also be included in the scope of the present invention.

The composition of the present invention has an excellent antiviral effect on a subject infected with virus, particularly DNA or RNA virus, and thus may be used to prevent or treat viral infection.

In the present invention, the "subject" is a subject that has or is suspected of having symptoms of viral infection, and thus is in need of ameliorating or treating viral infection or diseases caused by viral infection by inhibiting viral activity, etc., and it refers to any animals, including humans, that have been already infected with virus or are likely to be infected with virus.

In the present invention, the target virus against which the antiviral effect is exhibited may be a DNA virus or an RNA virus, more preferably an RNA virus. Among viruses, an RNA virus refers to a virus having single-stranded RNA or double-stranded RNA as a genetic material, and most RNA viruses have double-stranded RNA as a genetic material. RNA viruses that have RNA as a genetic material include those having (+) chain RNA that becomes mRNA, complementary (-) chain RNA, or double-stranded RNA as genes in virus particles, and those having only one RNA molecule, a virus having two identical RNA molecules (retrovirus), and a virus having eight different RNA molecules as genes (influenza virus). There is usually an RNA synthase (DNA synthase in the case of a retrovirus) that uses RNA as a template. RNA viruses have no RNA repair mechanism, and thus errors are highly likely to occur during replication. Such errors are likely to cause mutations in the genome. RNA virus is one of the major agents that cause viral diseases.

In the present invention, the virus may be a DNA virus or an RNA virus. In the present invention, the "RNA virus" means any virus that has RNA as a genetic material. For example, the RNA virus may belong to at least one selected from the group consisting of *Flaviviridae, Coronavirinae, Reoviridae, Picornaviridae, Caliciviridae, Togaviridae, Arenaviridae*, *Orthomyxoviridae*, *Paramyxoviridae, Bunyaviridae, Rhabdoviridae, Filoviridae, Astroviridae*, *Bornaviridae,* and *Arteriviridae*, and more preferably, may belong to *Flaviviridae* or *Coronavirinae.*

In the present invention, the *Flaviviridae* is a family of enveloped positive-strand RNA viruses that mainly infect mammals and birds, and these viruses are known to be primarily spread through arthropod vectors (mainly ticks and mosquitoes). The family gets its name from the yellow fever virus; *flavus* is Latin for "yellow", and yellow fever in turn was named because of its propensity to cause jaundice in victims. There are 89 species in the family divided among four genera. Diseases associated with the *Flavivirus* include yellow fever, Japanese encephalitis, West Nile virus, Saint Louis encephalitis, tick-borne encephalitis virus, dengue virus, and the like.

In the present invention, the dengue virus belongs to the genus *Flavivirus* of the family *Flaviviridae,* and is a positive single-stranded RNA virus, and there are Dengue serotypes 1, 2, 3, and 4. Dengue virus infections are caused by mosquitoes infected with dengue virus, and are classified into dengue fever (DF), dengue hemorrhagic fever (DHF), dengue shock syndrome (DSS), and the like depending on symptoms. Once infected with dengue virus, immunity against the same type of dengue virus is formed for a lifetime, but if re-infected with another type, immunity is not established, and symptoms can be aggravated. Dengue fever and dengue hemorrhagic fever have been reported to occur in a wide range of tropical and subtropical regions, including Asia, Central and South America, and Africa, and hundreds of millions of infected people occur annually. Furthermore, because the habitat environment of mosquitoes, which are vectors, has increased due to climate change, the possibility of infection in an epidemic area is very high, and thus the possibility of occurrence and spread of infected persons is increasing.

In the present invention, viruses of the *Coronavirinae* family are classified into four genera (alpha, beta, gamma, and delta), and are known as RNA viruses with a genome size of 27 to 32 kb, which infect the respiratory and digestive systems of humans and animals. They easily cause infection mainly by mucosal transmission and droplet transmission, and generally cause mild respiratory infections in humans, but rarely cause fatal infections, and they cause diarrhea in cattle and pigs, and respiratory diseases in chickens. Among coronaviruses, coronaviruses that use humans as hosts are classified as shown in Table 1 below (Korea Centers for Disease Control and Prevention, 2020). It has been reported that, of the four genera, alpha and beta infect humans and animals, while gamma and delta infect only animals.

**[Table 1]**

| Genus | Human-coronaviruses | Non-human infectious coronaviruses |
|---|---|---|
| Alpha coronaviruses | 229, NL63 | Porcine epidemic diarrhea virus (PEDV), (porcine) transmissible gastroenteritis virus (TGEV), canine coronavirus (CCoV), feline coronavirus (FCoV), Miniopterus bat coronavirus 1, Miniopterus bat coronavirus HKU8, Rhinolophus bat coronavirus HKU2, Scotophilus bat coronavirus 512 |
| Beta coronaviruses | OC43, HKU1, SARS-CoV, SARS-CoV2, MERS-CoV | Porcine hemagglutinating encephalomyelitis virus (PHEV), bovine coronavirus (BCoV), equine coronavirus (EqCoV), murine coronavirus (MuCoV), Tylonycteris bat coronavirus HKU4, Pipistrellus bat coronavirus HKU5, Rousettus bat coronavirus HKU9 |
| Gamma coronaviruses | - | Avian coronavirus, Beluga whale- coronavirus SW1 |
| Delta coronaviruses | - | Bulbul-coronavirus HKU11, Thrush-coronavirus HKU12, Munia-coronavirus HKU13 |

In particular, currently, coronaviruses that can infect humans are classified into seven types as shown in Table 2 below: types that cause colds (229E, OC43, NL63, and HKU1), and types that cause severe pneumonia (SARS-CoV, SARS-CoV-2, and MERS-CoV).

**[Table 2]**

| Virus name | Genus | Host | Symptoms |
|---|---|---|---|
| HCoV-229E | Alpha | Human | Mild respiratory symptoms |
| HCoV-NL63 | Alpha | Human | Mild respiratory symptoms |
| SARS-CoV | Beta | Human | Severe respiratory symptoms |
| MERS-CoV | Beta | Human | Severe respiratory symptoms |
| HCoV-OC43 | Beta | Human | Mild respiratory symptoms |
| HCoV-HKU1 | Beta | Human | Pneumonia symptoms |
| SARS-CoV-2 | Beta | Human | Mild respiratory symptoms, and shortness of breath in severe cases |

In the present invention, the "beta coronaviruses" belong to one of the four genera of the family *Coronaviridae* and correspond to a zoonotic infection. Known examples of the beta coronaviruses include severe acute respiratory syndrome virus (SARS; SARS-CoV), severe acute respiratory syndrome virus-2 (SARS-CoV-2), Middle East respiratory syndrome virus (MERS; MERS-CoV), human coronavirus OC43 (HCoV-OC43), human coronavirus HKU1 (HCoV-HKU1), and the like. In the present invention, the "severe acute respiratory syndrome virus-2 (SARS-CoV-2)" corresponds to an enveloped, positive-polar single-stranded RNA beta coronavirus belonging to the family *Coronaviridae.* Coronaviruses that infect humans historically include several common cold viruses, including hCoV-OC43, HKU, and 229E5. Sequence analysis of SARS-CoV-2 isolates suggests that the 30-kb genome encodes 14 open-reading frames (ORFs). At the 3' end of the viral genome, 13 ORFs are expressed from 9 predicted sub-genomic RNAs, and these include four structural proteins (spike (S), envelope (E), membrane (M) and nucleocapsid (N)) and nine putative accessory factors. Here, the nucleocapsid protein includes N1, N2 and N3 segments.

In the present invention, the coronaviruses include variants in which mutation has occurred naturally or artificially.

In the present invention, infectious diseases caused by the coronavirus may be coronavirus-induced enteritis, coronavirus-induced diarrhea, severe acute respiratory syndrome coronavirus (SARS), Middle East respiratory syndrome (MERS), or combinations thereof, but may include without limitation any disease that may be caused by coronavirus infection.

In the present invention, the infectious disease may be a respiratory disease, hepatitis, gastroenteritis or encephalitis caused by the viral infection.

The composition of the present invention may be used alone or in combination with conventional antiviral agents, or may be used in combination with biological agents such as antiserum.

Another embodiment of the present invention is directed to a composition for preventing, ameliorating or treating viral infection or infectious disease comprising a steroid sulfatase inhibitor as an active ingredient.

The composition of the present invention may be used for a pharmaceutical composition, a food composition, a food additive composition, a feed composition or a feed additive composition, without being particularly limited thereto.

In the present invention, the term "prevention" refers to any action that delays the progress of viral infection or multiplication by administering the composition of the present invention.

In the present invention, the compound or pharmaceutical composition may be in the form of capsules, tablets, granules, injections, ointments, powders, or beverages, and the pharmaceutical composition may be for administration to humans.

For use, the pharmaceutical composition of the present invention may be formulated in the form of oral preparations such as powders, granules, capsules, tablets, and aqueous suspensions, preparations for external use, suppositories, and sterile injectable solutions, according to the respective conventional methods, without being limited thereto. The pharmaceutical composition of the present invention may comprise pharmaceutically acceptable carriers. As the pharmaceutically acceptable carriers, a binder, a lubricant, a disintegrant, an excipient, a solubilizer, a dispersant, a stabilizer, a suspending agent, a colorant, a flavoring agent, and the like may be used for oral administration; a buffer, a preserving agent, a pain-relieving agent, a solubilizer, an isotonic agent, a stabilizer, and the like may be used for injection; and a base, an excipient, a lubricant, a preserving agent, and the like may be used for topical administration. In addition, the pharmaceutical composition of the present invention may be prepared in various dosage forms by being mixed with the pharmaceutically acceptable carriers as described above. For example, for oral administration, the pharmaceutical composition may be formulated in the form of tablets, troches, capsules, elixirs, suspensions, syrups, wafers, or the like. For injection, the pharmaceutical composition may be formulated in the form of unit dosage ampoules or in multiple-dosage forms. In addition, the pharmaceutical composition may be formulated into solutions, suspensions, tablets, capsules, sustained-release preparations, or the like.

Meanwhile, examples of carriers, excipients and diluents suitable for formulation include lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinyl pyrrolidone, water, methylhydroxy benzoate, propylhydroxy benzoate, talc, magnesium stearate, and mineral oil. In addition, the pharmaceutical composition of the present invention may further comprise a filler, an anticoagulant, a lubricant, a wetting agent, a fragrance, an emulsifier, a preservative, or the like.

The routes of administration of the compound or pharmaceutical composition according to the present invention include, but are not limited to, oral, intravenous, intramuscular, intra-arterial, intramedullary, intradural, intracardiac, transdermal, subcutaneous, intraperitoneal, intranasal, gastrointestinal, topical, sublingual and intrarectal routes. Oral or parenteral administration is preferred.

In the present invention, "parenteral" includes subcutaneous, transdermal, intravenous, intramuscular, intra-articular, intra-synovial, intrasternal, intradural, intralesional and intra-cranial injection or infusion techniques. The pharmaceutical composition of the present invention may also be formulated as suppositories for intrarectal administration.

The administration dose of the compound or pharmaceutical composition of the present invention may vary depending on various factors, including the activity of a specific compound used, the patient's age, body weight, general health, sex, diet, the time of administration, the route of administration, excretion rate, drug combination, and the severity of a particular disease to be prevented or treated. Although the dose of the pharmaceutical composition varies depending on the patient's condition and body weight, the severity of the disease, the form of drug, the route of administration, and the duration of administration, it may be appropriately selected by a person skilled in the art. The pharmaceutical composition may be administered at a dose of 0.0001 to 50 mg/kg/day or 0.001 to 50mg/kg/day. The pharmaceutical composition may be administered once or several times a day. The dose does not limit the scope of the present invention in any way. The pharmaceutical composition according to the present invention may be formulated as pills, sugar-coated tablets, capsules, liquids, gels, syrups, slurries, or suspensions.

In the present invention, the food composition may be prepared as various foods, for example, beverages, gums, teas, vitamin complexes, powders, granules, tablets, capsules, confectionery, cakes, bread and the like. In addition, it may also be used as a food additive composition necessary for the preparation of the food composition.

When the compound comprised as an active ingredient in the present invention is contained in the food composition, it may added in an amount of 0.1 to 50 wt% based on the total weight of the food composition. When the food composition is prepared as a beverage, there is no particular limitation, except that the beverage comprises the food composition at the indicated percentage. The beverage may additionally contain various flavorings or natural carbohydrates, like conventional beverages. Examples of the natural carbohydrates include monosaccharides such as glucose, disaccharides such as fructose, polysaccharides such as sucrose, conventional sugars such as dextrin, cyclodextrin or the like, and sugar alcohols such as xylitol, sorbitol, erythritol or the like. Examples of the flavorings include natural flavorings (thaumatin, stevia extracts, such as rebaudioside A, glycyrrhizin, etc.), and synthetic flavorings (saccharin, aspartame, etc.).

In addition, the food composition or food additive composition of the present invention may comprise various nutrients, vitamins, minerals (electrolytes), flavorings such as synthetic flavorings and natural flavorings, colorants, pectic acid and salts thereof, alginic acid and salts thereof, organic acids, protective colloidal thickeners, pH adjusting agents, stabilizers, preservatives, glycerin, alcohol, carbonizing agents that are used in carbonated beverages, etc.

These components may be used individually or in combination. Although the percentage of such additives is not of great importance, it is generally selected in the range of 0.1 to about 50 parts by weight based on 100 parts by weight of the food composition of the present invention.

When the compound of the present invention is comprised and used in the feed composition or feed additive composition, the composition may be prepared as 20 to 90% high concentrated liquid, power, or granule type. The feed additive composition may additionally comprise any one or more of organic acid, such as citric acid, fumaric acid, adipic acid, lactic acid, malic acid; phosphate, such as sodium phosphate, potassium phosphate, acid pyrophosphate, polyphosphate; or natural antioxidants, such as polyphenol, catechin, alpha-tocopherol, rosemary extract, vitamin C, green tea extract, licorice root extract, chitosan, tannic acid, phytic acid, etc.

In the present invention, when the compound is used as a feed, the composition may be formulated in a conventional feed form and may also comprise common feed ingredients. The feed additive and feed may additionally comprise grains, for example, powdered or pulverized wheat, oats, barley, corn and rice; vegetable protein feeds, for example, feeds based on rape, soybean and sunflower; animal protein feeds, for example, blood meal, meat meal, bone meal and fish meal; and sugar and dairy products, for example, various dry ingredients consisting of milk powder and whey powder, and may further comprise nutritional supplements, digestion- and absorption-enhancers, and growth promoters.

In the present invention, when the compound is used as the feed additive, it may be administered to animals alone or in combination with other feed additives selected from edible carriers. Further, the feed additive may be administered as a top dressing, or may be directly mixed with feeds, or may be easily administered to animals as oral dosage forms separately from feeds. When the feed additive is administered separately from feeds, the feed additive may be combined with a pharmaceutically acceptable edible carrier and prepared into an immediate-release formulation or sustained-release formulation, as well known in the art. Such edible carriers may be solid or liquid carriers, for example, corn starch, lactose, sucrose, soy flake, peanut oil, olive oil, sesame oil and propylene glycol. When solid carriers are used, the feed additive may be in the form of tablets, capsules, powders, troches or lozenges, or may be a non-dispersed top dressing. When liquid carriers are used, the feed additive may be in the form of a gelatin soft capsule, a syrup suspension, an emulsion, or a solution. The feed may comprise any protein-containing organic cereal flour that is commonly used to satisfy the dietary demand of animals. The protein-containing cereal flour may generally comprise corn, bean flour, or a corn/bean flour mix. In addition, the feed composition and feed additive composition may comprise, for example, a preservative, a stabilizer, a wetting agent, an emulsifier, or a solubilizer. In addition, the feed additive composition may be added to feeds by means of dipping, spraying or mixing for use.

In the composition for preventing, ameliorating or treating viral infection or infectious disease according to the present invention, definitions of the steroid sulfatase inhibitor, irosustat, virus classification, the pharmaceutically acceptable salt, etc. overlap with those described above with respect to the antiviral pharmaceutical composition, and thus will be omitted to avoid excessive complexity of the specification.

Still another embodiment of the present invention is directed to a method for inhibiting viral activity comprising administering a pharmaceutically effective amount of a composition comprising a steroid sulfatase inhibitor as an active ingredient to a subject in need of administration.

Yet another embodiment of the present invention is directed to a method for preventing or treating viral infection or infectious disease comprising administering a pharmaceutically effective amount of a composition comprising a steroid sulfatase inhibitor as an active ingredient to a subject in need of administration.

The steroid sulfatase inhibitor of the present invention may be at least one selected from the group consisting of irosustat, 2-(hydroxyphenyl) indole sulfate, 5-androstene-3,17-diol-3 sulfate, estrone-3-O-methylthiophosphonate (E1-3-MTP), estrone-3-O-sulfamate (EMATE), estradiol-3-O-sulfamate (E2MATE), 4-methylcoumarin 7-O-sulfamate (COUMATE), KW-2581, STX213, and morpholino, and may be, for example, irosustat, without being limited thereto.

In the present invention, descriptions of the steroid sulfatase inhibitor, irosustat, virus, viral infection or infectious disease, etc. are the same as those described above with respect to the antiviral pharmaceutical composition, and thus will be omitted to avoid excessive complexity of the present specification.

In the present invention, the term "administration" means providing a predetermined compound of the present invention to a subject by any suitable method.

In the present invention, the "subject" in need of administration may include both mammals and non-mammals. Here, examples of the mammals include, but are not limited to, humans, non-human primates such as chimpanzees, other ape or monkey species; livestock animals such as cattle, horses, sheep, goats, and pigs; domesticated animals such as rabbits, dogs or cats; laboratory animals, for example, rodents such as rats, mice, or guinea pigs. In addition, examples of the non-mammals in the present invention include, but are not limited to, birds or fish.

In the present invention, the formulation of the compound that is administered as described above is not particularly limited, and may be administered and may be administered as a solid formulation, a liquid formulation, or an aerosol formulation for inhalation. Specifically, the compound may be administered as solid form preparations which are intended to be converted, shortly before use, to liquid form preparations for oral or parenteral administration. For example, the compound may be formulated and administered as oral dosage forms, including powders, granules, capsules, tablets, and aqueous suspensions, preparations for external use, suppositories, and sterile injectable solutions, without being limited thereto.

In addition, in the present invention, pharmaceutically acceptable carriers may be additionally administered together with the compound of the present invention. Here, as the pharmaceutically acceptable carriers, a binder, a lubricant, a disintegrant, an excipient, a solubilizer, a dispersant, a stabilizer, a suspending agent, a colorant, a flavoring agent, and the like may be used for oral administration; a buffer, a preserving agent, a pain-relieving agent, a solubilizer, an isotonic agent, a stabilizer, and the like may be used for injection; and a base, an excipient, a lubricant, a preserving agent, and the like may be used for topical administration. In addition, the compound of the present invention may be prepared in various dosage forms by being mixed with the pharmaceutically acceptable carriers as described above. For example, for oral administration, the compound may be formulated in the form of tablets, troches, capsules, elixirs, suspensions, syrups, wafers, or the like. For injection, the compound may be formulated in the form of unit dosage ampoules or in multiple-dosage forms. In addition, the compound may be formulated into solutions, suspensions, tablets, capsules, sustained-release preparations, or the like.

Meanwhile, examples of carriers, excipients and diluents suitable for formulation include lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinyl pyrrolidone, water, methylhydroxy benzoate, propylhydroxy benzoate, talc, magnesium stearate, and mineral oil. In addition, the pharmaceutical composition of the present invention may further comprise a filler, an anticoagulant, a lubricant, a wetting agent, a flavoring agent, an emulsifier, a preservative, or the like.

The routes of administration of the compound according to the present invention include, but are not limited to, oral, intravenous, intramuscular, intra-arterial, intramedullary, intradural, intracardiac, transdermal, subcutaneous, intraperitoneal, intranasal, gastrointestinal, topical, sublingual and intrarectal routes. Oral or parenteral administration is preferred.

In the present invention, "parenteral" includes subcutaneous, transdermal, intravenous, intramuscular, intra-articular, intra-synovial, intrasternal, intradural, intralesional and intra-cranial injection or infusion techniques. The pharmaceutical composition of the present invention may also be formulated as suppositories for intrarectal administration.

In the present invention, the term "pharmaceutically effective amount" refers to a sufficient amount of an agent to provide a desired biological result. Said result may be reduction and/or alleviation of a sign, symptom, or cause of a disease, or any other desired alteration of a biological system. For example, an "effective amount" for therapeutic uses is the amount of the compound disclosed in the present invention, which is required to provide a clinically significant reduction in the disease. An appropriate "effective amount" in any individual case may be determined by one of ordinary skill in the art using routine experimentation. Thus, the expression "effective amount" generally refers to an amount in which an active substance has a therapeutic effect. In the case of the present invention, the active substance serves as both a steroid sulfatase inhibitor and an inhibitor of viral activity.

The administration dose of the compound of the present invention may vary depending on various factors, including the activity of a specific compound used, the patient's age, body weight, general health, sex, diet, the time of administration, the route of administration, excretion rate, drug combination, and the severity of a particular disease to be prevented or treated. Although the dose of the compound varies depending on the patient's condition and body weight, the severity of the disease, the form of drug, the route of administration, and the duration of administration, it may be appropriately selected by a person skilled in the art. The compound may be administered at a dose of 0.0001 to 100 mg/kg/day or 0.001 to 100 mg/kg/day. The pharmaceutical composition may be administered once or several times a day. The dose does not limit the scope of the present invention in any way. The compound according to the present invention may be formulated as pills, sugar-coated tablets, capsules, liquids, gels, syrups, slurries, or suspensions.

The compound of the present invention may be used alone or in combination with surgery, radiotherapy, hormone therapy, chemotherapy, and methods that use biological response modifiers.

Still yet another embodiment of the present invention is directed to a method for preventing, ameliorating or treating viral infection or infectious disease comprising administering a steroid sulfatase inhibitor to a subject.

In the present invention, the "subject" is a subject that has or is suspected of having symptoms of viral infection, and thus is in need of ameliorating or treating viral infection or diseases caused by viral infection by inhibiting viral activity, etc., and it refers to any animals, including humans, that have been already infected with virus or are likely to be infected with virus.

In the method for preventing, ameliorating or treating viral infection or infectious disease according to the present invention, definitions of the steroid sulfatase inhibitor, irosustat, virus classification, the pharmaceutically acceptable salt, etc. overlap with those described above with respect to the composition for preventing, ameliorating or treating viral infection or infectious disease, and thus will be omitted to avoid excessive complexity of the specification.

A further embodiment of the present invention is directed to a disinfectant or cleaning composition having inhibitory activity against virus, the composition comprising a steroid sulfatase inhibitor as an active ingredient.

Another further embodiment of the present invention is directed to a method for disinfecting or cleaning virus comprising contacting a steroid sulfatase inhibitor to an object.

In the present invention, the "disinfection" means to deactivate or kill pathogenic microorganisms or pathogenic viruses. Deactivation includes inhibiting pathogenic microorganism or pathogenic virus infection, survival, multiplication, spread, or any combination thereof.

In the present invention, the "cleaning" refers to a method used to facilitate or aid in contaminant removal, bleaching, microbial population reduction, rinsing, or any combination thereof.

In the present invention, the "object" includes any living or non-living objects (instruments, devices, clothing, tableware, etc.) or hard surfaces thereof that are to be prevented from being infected with viruses.

Since the steroid sulfatase inhibitor of the present invention has the ability to specifically kill viruses, the disinfectant composition or cleaning composition comprising the inhibitor according to the present invention may be effectively used as a disinfectant and a cleaner for hospital and health use to prevent hospital infection, and may be used as a general life disinfectant and cleaner and for disinfection and cleaning of various items such as tableware, food cooking equipment, inside buildings, drinking water, vehicles, aircrafts, and ships.

It is to be understood that, when the composition of the present invention is used as a disinfectant composition or a cleaning composition, it may also comprise a pharmacologically, veterinarily or agriculturally acceptable carrier or diluent.

In addition, the compound or composition of the present invention may be prepared in any phase such as a liquid phase, a solid phase, or a gas phase.

The compound or composition of the present invention may be applied to the human body itself or various instruments or devices used by humans, and when it is applied to the human body, it may be administered in any manner such as an oral or parenteral manner. In addition, the compound or composition may be formulated in various forms that may be effectively applied to the human body or various instruments or devices, and examples of the formulation include pharmaceutical formulations for oral administration such as tablets, pills, capsules, and powder formulations, or external preparations such as sprays, cleaning gels, liquids, creams, and ointments, or formulations applicable to instruments and devices.

In the present invention, the "contact" as a method of applying the compound or composition to an object may be a known disinfectant or cleaner application method appropriately selected depending on the nature of the object, the surrounding environment, etc., and may be performed by wiping, dipping, immersing, or spraying, preferably spraying, without being limited thereto.

In the disinfectant or cleaning composition of the present invention, definitions of the steroid sulfatase inhibitor, irosustat, virus classification, the pharmaceutically acceptable salt, etc. overlap with those described above, and thus will be omitted to avoid excessive complexity of the specification.

### Advantageous Effects

The composition comprising the steroid sulfatase inhibitor, which is provided in the present invention, is a safe substance without toxicity and side effects and has inhibitory activity against various viruses, and thus is capable of effectively preventing, ameliorating or treating viral infection or disease caused by viral infection.

### Brief Description of Drawings

FIG. 1 is a graph showing the results of measuring the mRNA expression level of *Ifnb1*, a type 1 interferon, through real-time qPCR analysis in one Example of the present invention.
FIG. 2 depicts graphs showing the results of measuring the mRNA expression levels of the antiviral genes *Mx1* and *Mx2,* which exhibit key effects of type 1 interferon, through real-time qPCR analysis in one Example of the present invention.
FIG. 3 is a graph showing the results of measuring viral titer through viral RNA extraction during a dengue virus infection experiment *in vitro* in one Example of the present invention.
FIG. 4 is a graph showing the results of measuring viral titer through viral RNA extraction during a severe acute respiratory syndrome viral infection experiment *in vivo* in one Example of the present invention.
FIGS. 5 and 6 respectively show the results of optical microscopic observation of the mouse lung tissue stained by the H & E staining method in one Example of the present invention, and the results of calculating the pathological score for the findings of inflammation in the lung.

### Best Mode

One embodiment of the present invention is directed to an antiviral pharmaceutical composition comprising a steroid sulfatase inhibitor as an active ingredient.

Another embodiment of the present invention is directed to a method for inhibiting viral activity comprising administering a pharmaceutically effective amount of a composition comprising a steroid sulfatase inhibitor as an active ingredient to a subject in need of administration.

Still another embodiment of the present invention is directed to a pharmaceutical composition for preventing or treating viral infection or infectious disease comprising a steroid sulfatase inhibitor as an active ingredient.

Yet another embodiment of the present invention is directed to a method for preventing or treating viral infection or infectious disease comprising administering a pharmaceutically effective amount of a composition comprising a steroid sulfatase inhibitor as an active ingredient to a subject in need of administration.

Still yet another embodiment of the present invention is directed to a food or food additive composition for preventing or ameliorating viral infection or infectious disease comprising a steroid sulfatase inhibitor as an active ingredient.

A further embodiment of the present invention is directed to a food or food additive composition for preventing or alleviating viral infection or infectious disease comprising a steroid sulfatase inhibitor as an active ingredient.

Another further embodiment of the present invention is directed to a disinfectant or cleaning composition having inhibitory activity against virus, the composition comprising a steroid sulfatase inhibitor as an active ingredient.

Still another further embodiment of the present invention is directed to a method for preventing or treating viral infection or infectious disease comprising administering a steroid sulfatase inhibitor to a non-human subject.

Yet another further embodiment of the present invention is directed to a method for disinfecting or cleaning virus comprising contacting a steroid sulfatase inhibitor to an object.

### Mode for Invention

Hereinafter, the present invention will be described in more detail with reference to examples. These examples are only for explaining the present invention in more detail, and it will be apparent to those skilled in the art that the scope of the present invention according to the subject matter of the present invention is not limited by these examples.

### Preparation Example 1: Preparation of irosustat

The compound (6-oxo-8,9,10,11-tetrahydro-7H-cyclohepta[c]chromen-3-yl)sulfamate (hereinafter referred to as "irosustat") represented by the following Formula 1 was purchased and prepared:

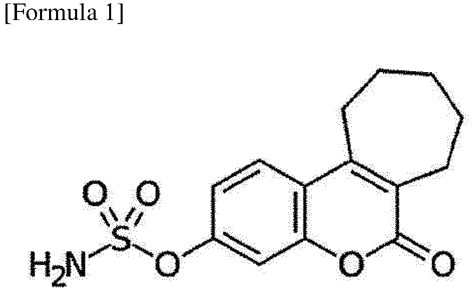

### Preparation Example 2: Preparation of mice

The inventors of the present invention purchased 8-week-old C57BL/6 male mice from Central Laboratory Animals and used the mice in the following experiments. Regarding laboratory environmental conditions, the mice were acclimatized for 1 week at a temperature of 21 ± 2°C and a humidity of 50 ± 10% with a 12-hour light/12-hour dark cycle while sufficient feed and water were provided. More specifically, 8-week-old C57BL/6 mice were orally administered vehicle or 10 mg/kg of irosustat once a day for 2 weeks and then euthanized, and the livers were harvested and used in the following experiments.

### Example 1: Evaluation of antiviral effect

In the following experiment, in order to evaluate the antiviral effect of irosustat, the livers harvested from the irosustat-administered mouse group and the vehicle group were homogenized with TissueLyser II (Qiagen), and then total RNA was isolated therefrom using an RNeasy mini kit (Qiagen). Thereafter, reverse transcription (RT) was performed on 2 µg of RNA with RevertAid RT Kit (EP0441, Thermo Fisher Scientific), and then real-time qPCR (qRT-PCR) was performed using SensiFAST SYBR Hi-ROX kit (BIO-92020, Bioline, London, UK). Based on the qPCR results, the mRNA expression level of the target gene was normalized to *Gapdh,* a house-keeping gene.

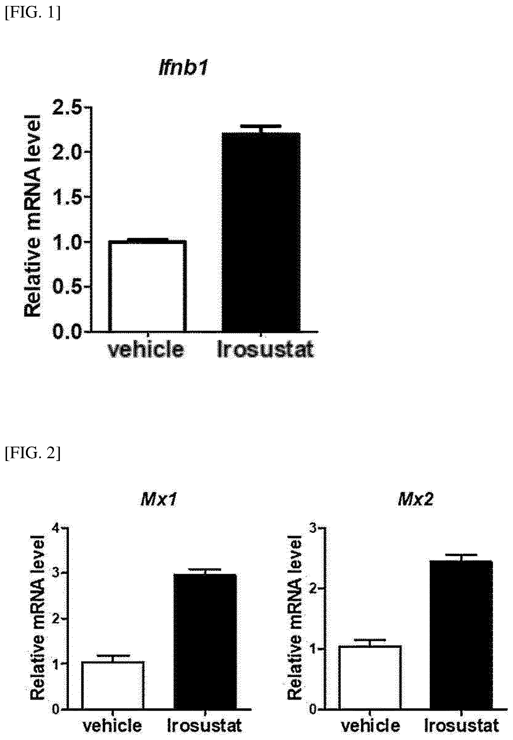

As a result, it was confirmed that, as shown in FIG. 1, the mRNA expression level of *Ifnb1* (interferon beta 1), a type 1 interferon, increased, and as shown in FIG. 2, the mRNA expression levels of *Mx1* and *Mx2* genes, which express the proteins Mx1 (MX Dynamin Like GTPase 1) and Mx2 (MX Dynamin Like GTPase 2) exhibiting a wide range of antiviral effects, increased.

It is known that type I interferons (IFNs) are proteins secreted mainly from virus-infected cells, and IFN-α and IFN-β are the most representative type I interferons in humans, which exhibit a wide range of antiviral effects (Nat Rev Immunol. 2015 Feb; 15(2):87-103). In addition, Mx1 protein (interferon-induced GTP-binding protein Mx1) and Mx2 protein (interferon-induced GTP-binding protein MX2) are both induced by type I interferons, and the interferon-induced Mx proteins correspond to markers known to accumulate in the cytoplasm in humans and to bind to invading viruses and induce viral death (Trends Microbiol. 2015 Mar; 23(3):154-63., Microbiol Mol Biol Rev. 2013 Dec; 77(4):551-66).

Therefore, the above results mean that the administration of irosustat upon viral infection exhibits an antiviral effect, suggesting that irosustat, which has already been proven safe as a therapeutic agent for metabolic diseases, may also be used to effectively prevent, ameliorate or treat viral infection or diseases caused by viral infection.

### Example 2: Evaluation of antiviral effect by in vitro experiment

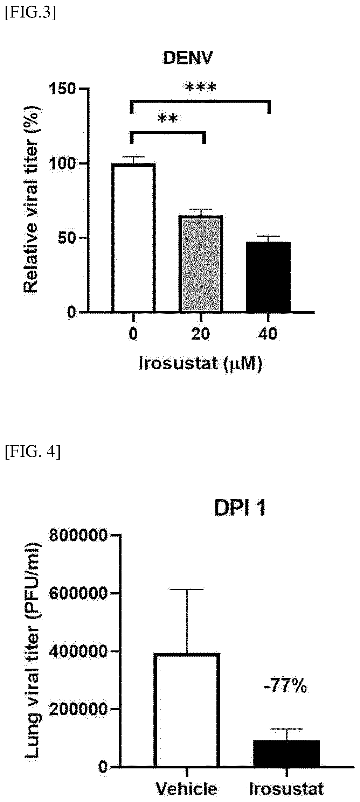

In order to evaluate the antiviral effect of irosustat *in vitro,* an additional experiment was performed in which Vero cells were infected with dengue virus. 16 hours before viral infection, cells were pre-treated with irosustat at a concentration of 0 µM, 20 µM or 40 µM, and 1 hour thereafter, the medium was replaced with a cell culture medium containing the drug. 48 hours after infection with the virus, the viral RNA titer of dengue virus in the cell culture medium was measured through qRT-PCR, and the results are shown in FIG. 3.

As a result of the experiment, it was confirmed that, when comparing the irosustat-treated group with the vehicle group, the viral RNA titer of dengue virus decreased in a manner dependent on the concentration of irosustat, and the virus titer decreased by about 50% or more upon treatment with irosustat at a concentration of 40 µM.

### Example 3: Evaluation of antiviral effect by in vivo experiment

To evaluate the antiviral effect of irosustat *in vivo,* 9-week-old K18-ACE2 TG mice were used in the experiment. Regarding laboratory environmental conditions, the mice were acclimatized for 1 week at a temperature of 21 ± 2°C and a humidity of 50 ± 10% with a 12-hour light/12-hour dark cycle while sufficient feed and water were provided. The mice were orally administered vehicle or 10 mg/kg of irosustat once a day from one day before infection with severe acute respiratory syndrome virus (SARS; SARS-CoV-2).

### 3.1 Virus titer measurement one day after virus infection

One day after virus infection, 3 vehicle mice and 4 irosustat-treated mice were euthanized and lung tissues were harvested therefrom. Viral titers in the lung tissues were measured by plaque assay, and the results are shown in FIG. 4.

As a result of the experiment, it could be confirmed that the viral titer in the lung tissue of the irosustat-treated group one day after virus infection was about 77% lower.

### 3.2 Calculation of inflammation score and edema score seven days after virus infection

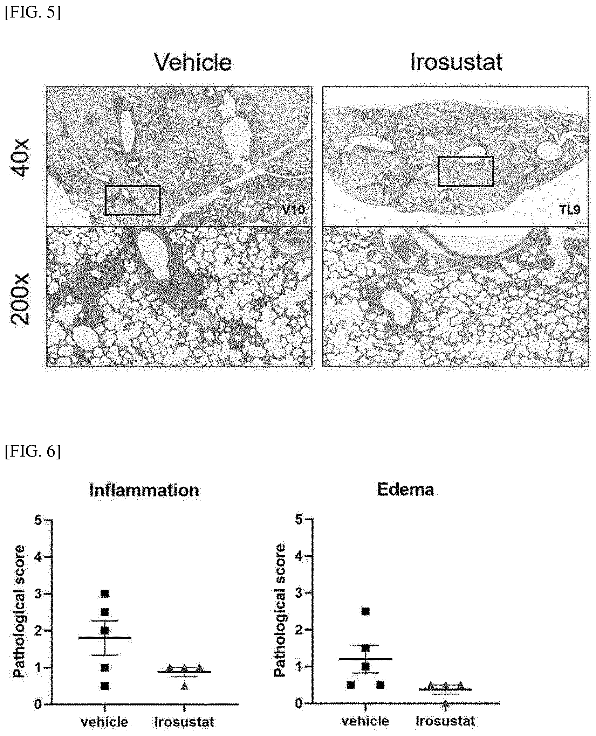

7 days after virus infection, the mice were euthanized and lung tissues were harvested therefrom, fixed in 10% neutral formalin, and then stained by the H & E staining method. The stained lung tissues were observed under an optical microscope, the pathological scores for inflammatory findings in the lungs were calculated, and the results are shown in FIGS. 5 and 6.

As a result of the experiment, it could be confirmed that, 7 days after virus infection, the number of inflammatory cells significantly decreased in the lung tissue of the irosustat-treated group, and both the calculated inflammation score and edema score were low in the lung tissue of the irosustat-treated group. These results mean that the symptoms of pneumonia caused by severe acute respiratory syndrome virus (SARS-CoV-2) are ameliorated by the drug irosustat, suggesting that irosustat may be applied for the treatment of coronavirus infection or diseases caused by viral infection.

Taken the above results together, the present inventors have verified the antiviral effect of the drug irosustat through various viral infection experiments, and thus it is expected that irosustat may be used as a therapeutic agent for infections or infectious diseases caused not only by coronavirus but also by various other viruses.

Although the present invention has been described in detail with reference to the specific features, it will be apparent to those skilled in the art that this description is only of a preferred embodiment thereof, and does not limit the scope of the present invention. Thus, the substantial scope of the present invention will be defined by the appended claims and equivalents thereto.

### Industrial Applicability

The composition of the present invention has inhibitory activity against various viruses, and thus is capable of effectively preventing, ameliorating or treating viral infection or diseases caused by viral infection.

## Claims

1. An antiviral pharmaceutical composition comprising a steroid sulfatase inhibitor as an active ingredient.

2. The composition according to claim 1, wherein the steroid sulfatase inhibitor is at least one selected from the group consisting of irosustat, 2-(hydroxyphenyl) indole sulfate, 5-androstene-3,17-diol-3 sulfate, estrone-3-O-methylthiophosphonate (E1-3-MTP), estrone-3-O-sulfamate (EMATE), estradiol-3-O-sulfamate (E2MATE), 4-methylcoumarin 7-O-sulfamate (COUMATE), KW-2581, STX213, and morpholino.

3. The composition according to claim 1, comprising a salt, hydrate or solvate form of the steroid sulfatase inhibitor.

4. The composition according to claim 1, wherein the virus is DNA virus or RNA virus.

5. The composition according to claim 4, wherein the virus belongs to at least one selected from the group consisting of *Flaviviridae, Coronavirinae, Reoviridae, Picornaviridae, Caliciviridae, Togaviridae, Arenaviridae*, *Orthomyxoviridae, Paramyxoviridae, Bunyaviridae, Rhabdoviridae, Filoviridae, Astroviridae*, *Bornaviridae*, and *Arteriviridae.*

6. The composition according to claim 5, wherein the virus belongs to *Flaviviridae* or *Coronavirinae.*

7. A pharmaceutical composition for preventing or treating viral infection or infectious disease comprising a steroid sulfatase inhibitor as an active ingredient.

8. The composition according to claim 7, wherein the steroid sulfatase inhibitor is at least one selected from the group consisting of irosustat, 2-(hydroxyphenyl) indole sulfate, 5-androstene-3,17-diol-3 sulfate, estrone-3-O-methylthiophosphonate (E1-3-MTP), estrone-3-O-sulfamate (EMATE), estradiol-3-O-sulfamate (E2MATE), 4-methylcoumarin 7-O-sulfamate (COUMATE), KW-2581, STX213, and morpholino.

9. The composition according to claim 7, comprising a salt, hydrate or solvate form of the steroid sulfatase inhibitor.

10. The composition according to claim 7, wherein the virus is DNA virus or RNA virus.

11. The composition according to claim 10, wherein the virus belongs to at least one selected from the group consisting of *Flaviviridae, Coronavirinae, Reoviridae, Picornaviridae, Caliciviridae, Togaviridae, Arenaviridae*, *Orthomyxoviridae, Paramyxoviridae, Bunyaviridae, Rhabdoviridae, Filoviridae, Astroviridae*, *Bornaviridae*, and *Arteriviridae.*

12. The composition according to claim 11, wherein the virus belongs to *Flaviviridae* or *Coronavirinae.*

13. A food or food additive composition for preventing or ameliorating viral infection or infectious disease comprising a steroid sulfatase inhibitor as an active ingredient.

14. The composition according to claim 13, wherein the steroid sulfatase inhibitor is at least one selected from the group consisting of irosustat, 2-(hydroxyphenyl) indole sulfate, 5-androstene-3,17-diol-3 sulfate, estrone-3-O-methylthiophosphonate (E1-3-MTP), estrone-3-O-sulfamate (EMATE), estradiol-3-O-sulfamate (E2MATE), 4-methylcoumarin 7-O-sulfamate (COUMATE), KW-2581, STX213, and morpholino.

15. The composition according to claim 13, comprising a salt, hydrate or solvate form of the steroid sulfatase inhibitor.

16. A feed or feed additive composition for preventing or ameliorating viral infection or infectious disease comprising a steroid sulfatase inhibitor as an active ingredient.

17. The composition according to claim 16, wherein the steroid sulfatase inhibitor is at least one selected from the group consisting of irosustat, 2-(hydroxyphenyl) indole sulfate, 5-androstene-3,17-diol-3 sulfate, estrone-3-O-methylthiophosphonate (El-3-MTP), estrone-3-O-sulfamate (EMATE), estradiol-3-O-sulfamate (E2MATE), 4-methylcoumarin 7-O-sulfamate (COUMATE), KW-2581, STX213, and morpholino.

18. The composition according to claim 16, comprising a salt, hydrate or solvate form of the steroid sulfatase inhibitor.

19. A disinfectant or cleaning composition having inhibitory activity against viruses, the composition comprising a steroid sulfatase inhibitor as an active ingredient.

20. The composition according to claim 19, wherein the steroid sulfatase inhibitor is at least one selected from the group consisting of irosustat, 2-(hydroxyphenyl) indole sulfate, 5-androstene-3,17-diol-3 sulfate, estrone-3-O-methylthiophosphonate (E1-3-MTP), estrone-3-O-sulfamate (EMATE), estradiol-3-O-sulfamate (E2MATE), 4-methylcoumarin 7-O-sulfamate (COUMATE), KW-2581, STX213, and morpholino.

21. The composition according to claim 19, comprising a salt, hydrate or solvate form of the steroid sulfatase inhibitor.

22. A method for preventing or treating viral infection or infectious disease comprising administering a steroid sulfatase inhibitor to a non-human subject.

23. The method according to claim 22, the steroid sulfatase inhibitor is at least one selected from the group consisting of irosustat, 2-(hydroxyphenyl) indole sulfate, 5-androstene-3,17-diol-3 sulfate, estrone-3-O-methylthiophosphonate (E1-3-MTP), estrone-3-O-sulfamate (EMATE), estradiol-3-O-sulfamate (E2MATE), 4-methylcoumarin 7-O-sulfamate (COUMATE), KW-2581, STX213, and morpholino.

24. A method for disinfecting or cleaning virus comprising contacting a steroid sulfatase inhibitor to an object.

25. The method according to claim 24, the steroid sulfatase inhibitor is at least one selected from the group consisting of irosustat, 2-(hydroxyphenyl) indole sulfate, 5-androstene-3,17-diol-3 sulfate, estrone-3-O-methylthiophosphonate (E1-3-MTP), estrone-3-O-sulfamate (EMATE), estradiol-3-O-sulfamate (E2MATE), 4-methylcoumarin 7-O-sulfamate (COUMATE), KW-2581, STX213, and morpholino.
